(19) Europäisches Patentamt
European Patent Office
Office européen des brevets

(11) Veröffentlichungsnummer : **0 336 202 B1**

(12) # EUROPÄISCHE PATENTSCHRIFT

(45) Veröffentlichungstag der Patentschrift :
**06.05.92 Patentblatt 92/19**

(51) Int. Cl.$^5$ : **C07C 249/00**

(21) Anmeldenummer : **89105009.8**

(22) Anmeldetag : **21.03.89**

(54) **Verfahren zur Herstellung von 4-Halo-3-oxo-2-alkoxyiminobuttersäureestern.**

(30) Priorität : **24.03.88 DE 3809845**

(43) Veröffentlichungstag der Anmeldung :
**11.10.89 Patentblatt 89/41**

(45) Bekanntmachung des Hinweises auf die Patenterteilung :
**06.05.92 Patentblatt 92/19**

(84) Benannte Vertragsstaaten :
**AT BE CH DE ES FR GB GR IT LI LU NL SE**

(56) Entgegenhaltungen :
**EP-A- 0 160 563**
**EP-A- 0 191 507**
**EP-A- 0 246 603**
**DE-A- 2 900 961**
**US-A- 4 480 120**

(73) Patentinhaber : **HOECHST AKTIENGESELLSCHAFT**
**Postfach 80 03 20**
**W-6230 Frankfurt am Main 80 (DE)**

(72) Erfinder : **Ritter, Eberhard, Dr.**
**Nordring 74**
**W-6082 Mörfelden-Walldorf (DE)**
Erfinder : **Krieg, Claus-Peter, Dr.**
**Im Mainfeld 42**
**W-6000 Frankfurt am Main 71 (DE)**
Erfinder : **Keil, Detlev, Dr.**
**Kirschgartenstrasse 12**
**W-6238 Hofheim am Taunus (DE)**

EP 0 336 202 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

4-Halo-3-oxo-2-alkoxyimino-buttersäureester der allgemeinen Formel

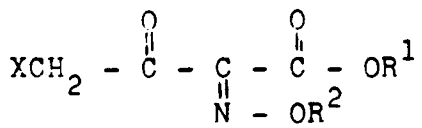

in der $R^1$ und $R^2$ für Alkyl, vorzugsweise $C_1$-$C_4$-Alkyl, steht, insbesondere $R^1$ für Ethyl und $R^2$ für Methyl, und X Halogen, vorzugsweise Chlor oder Brom, bedeutet, sind Zwischenprodukte für die Herstellung von Seitenkettensäuren, die einen wichtigen Baustein innerhalb der Synthese von Cephalosporin-Antibiotika darstellen. Sie werden gewöhnlich mit Thioharnstoff einer Hantzsch-Cyclisierung unterworfen. Nach Verseifung des Esters wird dann die Säure mit 7-Aminocephalosporansäure oder einem ihrer Derivate zum antibiotisch wirksamen Säureamid umgesetzt.

Ein möglicher Weg zur Synthese dieser Vorprodukte ist die Methylierung von 4-Halo-3-oxo-2-hydroxyimino-buttersäureestern mit Diazomethan oder Dialkylsulfaten (DE-OS 27 02 501, DE-OS 27 37 504, DE-OS 28 06 226 und DE-PS 27 13 272). Dieser Reaktionsweg leidet unter der schlechten Zugänglichkeit des Edukts.

Durchgesetzt hat sich daher die Halogenierung von 3-Oxo-2-methoxyimino-buttersäureestern, wofür verschiedene Verfahren bekannt sind. Alle bekannten Umsetzungen führen die Reaktion - gleich mit welchem Halogenierungsagens - in einem inerten Lösungsmittel durch. Vorgeschlagen wurden u.a. Aceton, Diethylether, Ameisensäure, Eisessig, Dimethylformamid und Tetrahydrofuran (EP 0 007 633, EP 0 049 539, EP 0 191 507). Die größte Verbreitung erlangte Methylenchlorid.

Als Halogenierungsmittel kamen die Halogene selbst, Sulfurylhalogenid, N-Halosuccinimid und Pyridinium-Perhalide zum Einsatz (EP 0 007 633). Von den genannten Halogenierungsmitteln haben vor allem Sulfurylchlorid zur Synthese der Chlorverbindung (X = Chlor) und elementares Brom zur Darstellung der analogen Bromverbindung breite Anwendung gefunden. Die Bromierung im Methylenchlorid dauert in der Regel eine Stunde bei Raumtemperatur, während die Chlorierung mit Sulfurylchlorid im selben Lösungsmittel 4 bis 10 Stunden bei 35 bis 42°C benötigt (EP 007 633, EP 0 059 539, EP 0 191 507, JA 56/100 772, US 4 480 120) und damit erheblich langsamer verläuft. Die Bromierung in Methylenchlorid war daher die für die Praxis interessantere Arbeitsweise.

Im Zuge zunehmender Sensibilität für den Umweltschutz und den Schutz des Personals vor dem Kontakt mit gesundheitsschädlichen Chemikalien erweist sich die Emission von Lösungsmitteldämpfen bei den Standardverfahren als problematisch. Würde es darüberhinaus gelingen, die Chlorierung ähnlich schnell wie die Bromierung zu machen, könnte das als sehr giftig eingestufte Brom substituiert werden.

Aufgabe war es daher, eine Alternative zu Methylenchlorid zu finden, um dieses kritische Lösungsmittel nicht mehr einsetzen zu müssen. Grundsätzlich schien der Einsatz eines Lösungsmittels aber nötig zu sein, um einen kontrollierten und selektiven Ablauf der Reaktion sicherzustellen. Zugleich sollte das neue Verfahren die Möglichkeit bieten, mit vergleichbaren Ausbeuten und in vergleichbaren Zeiten statt zu bromieren zu chlorieren.

Zunächst wurde die Eignung anderer, gewerbehygienisch unbedenklicher Lösungsmittel untersucht. Als dies nicht zum gewünschten Erfolg führte, wurde überraschend festgestellt, daß die Halogenierung auch ohne Einsatz eines Lösungsmittels mit guten Ausbeuten durchführbar ist.

Das erfindungsgemäße Verfahren ist also dadurch gekennzeichnet, daß man auf das flüssige Substrat eines 3-Oxo-2-alkoxyimino-buttersäureesters der allgemeinen Formel I

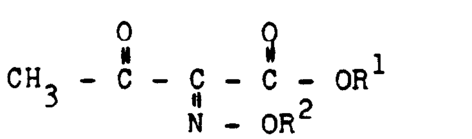

(I)

in der $R^1$ und $R^2$ für $C_1$-$C_4$-Alkyl stehen, elementares Halogen direkt, ohne Verwendung eines Lösungsmittels einwirken läßt und so einen 4-Halo-3-oxo-2-alkoxyimino-buttersäureester der allgemeinen Formel II

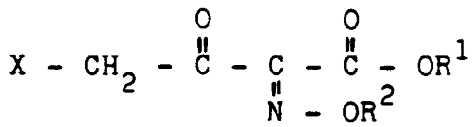

(II)

in der $R^1$ und $R^2$ die vorstehende Bedeutung besitzen und X für Halogen steht, erhält.

Im Hinblick auf die bekannte größere Bedeutung der syn-Verbindungen bei den Cephalosporinantibiotika sind auch erfindungsgemäß Verbindungen bevorzugt, in denen sie $R^2O$-Gruppe in syn-Stellung steht.

Als Bedeutungen für $R^1$ und $R^2$ seien genannte Methyl, Ethyl, n-Propyl, Isopropyl, n-Butyl, sec.-Butyl oder tert.-Butyl. Bevorzugt sind jedoch Verbindungen der allgemeinen Formeln I und II, in denen $R^1$ für Ethyl und $R^2$ für Methyl stehen.

Für X kommen vorzugsweise in Betracht Chlor, Brom und Jod, insbesondere Chlor und Brom.

Die Herstellung der Ester der allgemeinen Formel I kann nach dem Verfahren der DE-OS 27 02 501 erfolgen.

Das erfindungsgemäße Verfahren erwies sich als besonders geeignet für die technische Bromierung von 3-Oxo-2-alkoxyimino-buttersäureestern. Bei der Chlorierung mußten die experimentellen Details etwas verändert werden, was entgegen der Erwartung nicht mit Einbußen bei der Selektivität erkauft werden mußte.

Das erfindungsgemäße Verfahren läßt sich beispielsweise folgendermaßen durchführen.
Als Ausgangsmaterial wird ein Ester der allgemeinen Formel I in technischer Qualität oder destillativ gereinigt in einem Doppelmantel-Rührgefäß aus säurebeständigem Material, das mit einem Innenthermometer ausgestattet ist, vorgelegt. Für die Chlorierung handelt es sich vorteilhaft um ein Druckgefäß.

Bei der Bromierung werden etwa 0,5 bis 0,8, vorzugsweise 0,6 mol Brom je mol Ausgangsverbindung benötigt. Bei etwa 15 - 40°C, bevorzugt bei 20°C werden etwa 5 - 15 %, bevorzugt 10 % der benötigten Brommenge mit oder ohne, vorzugsweise ohne Rühren zugegeben. Durch die Entfärbung der Reaktionslösung und eine Temperaturerhöhung, beispielsweise um 6 - 10°C, ist der Reaktionsbeginn zu erkennen. Danach wird auf etwa 10 - 25°C, bevorzugt auf 15°C gekühlt und der Rest Brom unter Rühren so schnell zugesetzt, daß die Innentemperatur konstant bleibt. Der Reaktionsfortgang wird im HPLC beobachtet und die Reaktion bei beginnender Bildung des dibromierten Produkts abgebrochen. Der gebildete Bromwasserstoff wird etwa 30 Minuten lang mit Stickstoff ausgetrieben. In der Praxis kann das Produkt ohne weitere Reinigung, die beispielsweise durch Destillation möglich wäre, in die nächste Reaktionsstufe eingesetzt werden.

Bei der Chlorierung ist das Reaktionsgefäß zusätzlich mit einem Gaseinleitungsrohr auszustatten. In Apparaturen für größere Ansätze sind die Gasdurchtrittsöffnungen so zu wählen, daß zur Erzielung kleiner Blasen Gasdurchtrittsgeschwindigkeiten von etwa 5 - 50 m/s, vorteilhaft 10 - 50 m/s, bevorzugt 20 - 30 m/s bei einem Chlorstrom erreicht werden, der es gestattet, die freiwerdende Reaktionswärme noch abzuführen. Als Zusätzliche Sicherheitseinrichtung eignet sich eine Differenzdruckmessung zwischen Chlorvorratsbehälter und Reaktionskessel, die mit einem Schnellschlußventil gekoppelt ist.

Das Ausgangsmaterial wird auf etwa 30 - 40°C erwärmt, dann wird mit der Chloreinleitung begonnen. Nach Zugabe von etwa 5 - 15 %, vorteilhaft 10% der Gesamt-Chlormenge springt die Reaktion an, was am Termperaturanstieg erkennbar ist. Tritt dieser nicht ein, werden nach Unterbrechung der Einleitung dem Ausgangsmaterial etwa 0,1 - 2 %, vorteilhaft 0,1 - 1 %, bevorzugt 0,1 % konzentrierte Schwefelsäure oder eine andere wasserfreie Säure (Salzsäuregas, Essigsäure u.ä.) als Katalysator zugesetzt.

Nach dem Reaktionsstart wird bei etwa 30 - 70°C, bevorzugt 40 - 50°C, insbesondere 50° die Chloreinleitung fortgesetzt. Je mol organisches Substrat werden insgesamt etwa 1 - 1,4 mol, vorteilhaft 1,2 mol Chlor so schnell eingeleitet, daß die Innentemperatur gehalten werden kann.

Um in jedem Fall vollständigen Chlorumsatz zu erzwingen, kann die Einleitung so erfolgen, daß Chlor bis zum Ansprechen der Differenzdruckmessung zugeführt wird und daß danach das als zweites Reaktionsprodukt entstandene Salzsäuregas in einen mit z.B. 16 %iger Natronlauge betriebenen Gaswäscher abgelassen wird. Gegebenenfalls sind diese Schritte zu wiederholen bis alles Chlor eingeleitet ist. Vorteilhaft ist es jedoch, das HCl-Gas bei etwa 1 - 6 bar, vorteilhaft 2 - 6 bar, bevorzugt 3 bar Kesseldruck kontinuierlich abströmen zu lassen. Auch so lassen sich Chlorumsätze von über 99,5 % erzielen.

Das chlorierte Produkt kann ohne weitere Reinigung in die nächste Synthesestufe eingesetzte werden.

Die Anwendung der Erfindung führt zu einer verbesserten Synthese eines wichtigen Bausteins für hochwirksame Cephalosporin-Antibiotika. Da kein Lösungsmittel mehr benötigt wird, wird die Raum-Zeit-Ausbeute wesentlich erhöht. Probleme mit Lösungsmitteldämpfen, die schwierig aus der Abluft zu entfernen sind, treten nicht mehr auf. Da nach Umstellung der bisher üblichen Halogenierungsmethode auf das erfindungsgemäße Verfahren keine Synthesestufe der Antibiotika-Seitenkette mehr in organischen Medien abläuft, braucht keine

Lösungsmittelrückgewinnung oder -entsorgung mehr betrieben zu werden. Durch die Anwendung von erhöhtem Druck und erhöhter Temperatur wird die Geschwindigkeit der Chlorierung soweit gesteigert, daß sie in einer der Bromierung vergleichbaren Zeit abläuft.

Es war nicht vorauszusehen, daß das erfindungsgemäße Verfahren mit den aufgezeigten Vorteilen in so einfacher Weise durchführbar sein würde.

Die Verbindungen der allgemeinen Formel I werden nach den bisher üblichen Verfahren mit Thioharnstoff zum entsprechenden Thiazolester umgesetzt, der als Feststoff isoliert und danach alkalisch zur Seitenkettensäure verseift wird. Die erhaltene Säure erreicht Qualität und Reinheit des üblichen Standards nach den bisherigen Verfahren. Die Amide dieser Säure mit 7-Aminocephalosporansäure oder einem ihrer Derivate stellen die therapeutisch sehr wirksamen Cephalosporin-Antibiotika dar.

Die folgenden Beispiele dienen zur weiteren Erläuterung der Erfindung, schränken sie jedoch nicht darauf ein.

## Beispiele

### Beispiel 1

4-Brom-3-oxo-2-syn-methoxyimino-buttersäureethylester

In einem Rührgefäß mit Innenthermometer werden 100 ml technischer 3-Oxo-2-syn-methoxyimino-buttersäureethylester vorgelegt. Bei Raumtemperatur werden zunächst 2 ml elementares Brom zugegeben. Das Anspringen der Reaktion ist an der Entfärbung und an einem Temperaturanstieg um 6 - 10°C erkennbar. Während dieser Induktionsperiode wird nicht gerührt.

Anschließend wird auf 15°C abgekühlt und 14 - 17 ml Brom werden so zudosiert, daß die Innentemperatur 20°C nicht überschreitet. Der Zeitbedarf dafür beträft bei Wasserkühlung etwa 30 Minuten. Die Reaktion wird abgebrochen, wenn im HPLC vollständiger Umsatz der organischen Komponente festgestellt wird. Der gelöst verbliebene Bromwasserstoff wird 30 Minuten lang mit Stickstoff ausgetrieben.

### Beispiel 2

4-Chlor-3-oxo-2-syn-methoxyimino-buttersäureethylester

In einem Doppelmantel-Rührgefäß mit Innenthermometer werden 300 ml technischer 3-Oxo-2-syn-methoxyimino-buttersäureethylester bei 30°C vorgelegt. Über ein Einleitrohr mit Glasfritte werden in 7 Stunden 160 g Chlor bei Normaldruck eingeleitet. Die Innentemperatur wird auf 32 - 35°C thermostatisiert. Dabei kann die Einleitgeschwindigkeit zu Beginn etwa 8 - 10 l/h, gegen Reaktionsende noch 1 l/h Chlor betragen, ohne daß größere Chlormengen mit dem entweichenden Salzsäuregas in den mit Natronlauge betriebenen Absorber gelangen.

Der Reaktionsfortschritt kann mittels HPLC verfolgt werden. Nach Einleitung des gesamten Chlors wird 15 Minuten Stickstoff durch das Tauchrohr eingeleitet und das gelöste Salzsäuregas gestrippt. Man erhält ca. 390 g Rohprodukt, das bei Raumtemperatur lagerstabil ist und unmittelbar weiter verarbeitet werden kann.

Bei einer Aufreinigung destilliert es bei einem Druck von 1 mbar in einem Siedebereich von 92 - 96°C über. Das Destillat besitzt folgendes $^1$NMR-Spektrum ($CDCl_3$, 400 MHz):

$\delta$ (ppm):4,59 (s, $CH_2Cl$);
4,37 (q, 7,2 Hz, $C\underline{H}_2CH_3$);
4,13 (s, $OCH_3$);
1,35 (t, 7,2 Hz, $CH_2C\underline{H}_3$).

### Beispiel 3

4-Chlor-3-oxo-2-syn-methoxyimino-buttersäureethylester

In einem 250 l Email-Druckkessel mit der beschriebenen Ausrüstung werden 111 kg technischer 3-Oxo-2-syn-methoxyimino-buttersäureethylester bei 40°C vorgelegt. 42,8 kg Chlor werden so schnell eingeleitet, daß die Innentemperatur durch Solekühlung bei 50°C gehalten werden kann. Der minimale Zeitbedarf liegt bei 1,5 Stunden. Ist dabeinach Einleitung von 3,9 kg Chlor die Reaktion noch nicht angesprungen, was an der Wärmeentwicklung erkennbar ist, muß abgebrochen und nach Zugabe von 0,1 % Schwefelsäure neu gestartet werden. Fällt die Reaktortemperatur durch zu starkes Kühlen unter 40°C, darf die Einleitung erst nach Aufheizen

des Kesselinhalts fortgesetzt werden.

Durch ein Überströmventil wird der Kesselüberdruck bei 3 bar gehalten. Abströmendes Gas wird in einem Wäscher, in dem 120 l 16 %ige Natronlauge umgepumpt werden, geleitet. Man erhält etwa 130 kg Rohprodukt, das ohne Strippen und weitere Reinigung weiter umgesetzt wird.

## Patentansprüche

1. Verfahren zur Herstellung von 4-Halo-3-oxo-2-alkoxyimino-buttersäureestern durch Halogenierung von 3-Oxo-2-alkoxyimino-buttersäureestern, dadurch gekennzeichnet, daß man aus das flüssige Substrat eines 3-Oxo-2-alkoxyimino-buttersäureesters der allgemeinen Formel I

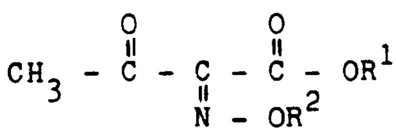 (I)

in der $R^1$ und $R^2$ für $C_1$-$C_4$-Alkyl stehen, elementares Halogen direkt, ohne Verwendung eines Lösungsmittels einwirken läßt und so einen 4-Halo-3-oxo-2-alkoxyimino-buttersäureester der allgemeinen Formel II

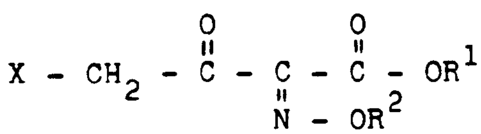 (II)

in der $R^1$ und $R^2$ die vorstehende Bedeutung besitzen und X für Halogen steht, erhält.

2. Verfahren gemäß Anspruch 1, dadurch gekennzeichnet, daß die $R^2$O-Gruppe in syn-Position steht.

3. Verfahren gemäß Ansprüchen 1 und 2, dadurch gekennzeichnet, daß $R^1$ für Ethyl und $R^2$ für Methyl steht.

4. Verfahren gemäß Ansprüchen 1 bis 3, dadurch gekennzeichnet, daß als Halogen elementares Chlor oder Brom eingesetzt wird.

## Claims

1. A process for the preparation of 4-halo-3-oxo-2-alkoxy-iminobutyric esters by halogenation of 3-oxo-2-alkoxy-iminobutyric esters, which comprises exposing the liquid substrate of a 3-oxo-2-alkoxyiminobutyric ester of the general formula I

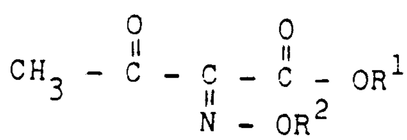 (I)

in which $R^1$ and $R^2$ represent $C_1$-$C_4$ alkyl, to the action of elemental halogen directly, wihtout using a solvent, and thus obtaining a 4-halo-3-oxo-2-alkkoxy-iminobutyric ester of the general formula II

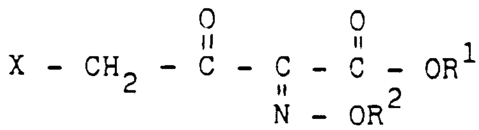 (II)

in which $R^1$ and $R^2$ have the previous meaning, and X represents halogen.

2. The process as claimed in claim 1, wherein the $R^2O$ group is in the syn position.

3. The process as claimed in claim 1 and 2, wherein $R^1$ represents ethyl and $R^2$ represents methyl.

4. The process as claimed in claim 1 and 3, wherein elemental chlorine or bromine is employed as halogen.

## Revendications

1. Procédé pour la préparation d'esters d'acide 4-halogéno-3-oxo-2-alcoxymino-butyrique par halogénation d'esters d'acide 3-oxo-2-alcoxyimino-butyrique, caractérisé en ce que l'on fait agir directement, sans utilisation d'un solvant, un halogène élémentaire sur le substrat liquide d'un ester d'acide 3-oxo-2-alcoxyimino-butyrique, de formule générale I

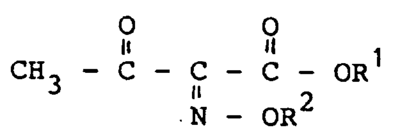

(I)

dans laquelle $R^1$ et $R^2$ représentent un groupe alkyle en $C_1$-$C_4$, et on obtient ainsi un ester d'acide 4-halogéno-3-oxo-2-alcoxyimino-butyrique de formule générale II

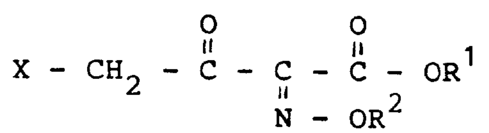

(II)

dans laquelle $R^1$ et $R_2$ ont les significations précédentes et X représente un halogène.

2. Procédé selon la revendication 1, caractérisé en ce que le groupe $R^2O$ est en position syn.

3. Procédé selon la revendication 1 ou 2, caractérisé en ce que $R^1$ représente le groupe éthyle et $R^2$ représente le groupe méthyle.

4. Procédé selon l'une quelconque des revendications 1 à 3, caractérisé en ce que, en tant qu'halogène on utilise le chlore ou le brome élémentaire.